# EUROPEAN PATENT SPECIFICATION

(11) **EP 3 224 322 B1**
(45) Date of publication and mention of the grant of the patent: **06.01.2021**
(21) Application number: 15807752.9
(22) Date of filing: 23.11.2015
(51) Int. Cl.: C09C 1/00, A61K 8/02, A61K 8/19, A61Q 1/06, A61Q 1/08, A61Q 1/10, A61Q 1/12, A61Q 3/02

(54) **CARBON BLACK IN EFFECT PIGMENTS**
RUSS IN EFFEKTPIGMENTEN
NOIR DE CARBONE DANS PIGMENTS À EFFET

(30) Priority: 24.11.2014 US 201462083541 P; 22.12.2014 US 201414579032
(43) Date of publication of application: 04.10.2017
(73) Proprietor: BASF Corporation, Florham Park, NJ 07932 (US)
(72) Inventor: PONCE, Lizzabeth, Lake Jackson, TX 77566 (US); JOHNSON, Geoffrey, Wappingers Falls, NY 12590 (US); CERCE, Louis R., Garrison, NY 10524 (US); JONES, Steven, Budd Lake, NJ 07828 (US); ZIMMERMANN, Curtis, Cold Spring, NY 10516 (US); MOHR, Benjamin Georg Robert, MI 48009 Birmingham (US); HOFFSTEAD-FORDYCE, Charmain, Walden, NY 12586 (US)
(74) Representative: Altmann, Andreas
(86) International application number: PCT/US2015/062183
(87) International publication number: WO 2016/085874

(56) References cited:
- EP-A1- 1 028 146
- WO-A1-01/29137
- WO-A2-2004/104109
- US-A- 4 076 551
- US-A- 5 286 291
- US-B1- 6 572 595

## Description

### FIELD OF THE INVENTION

This application is directed to effect pigments containing carbon black and methods of producing these pigments.

### BACKGROUND

Effect pigments (sometimes also called gloss pigments, lustrous pigments, pearlescent pigments, interference pigments, or color variable pigments) are well known in the art. A widely used type of effect pigment includes mica platelets coated with metallic oxides, such as titanium dioxide. A thin titanium dioxide (TiO₂) coating produces a pearl-like or silvery luster, with the color produced by this thin layer of TiO₂ being a function of optical thickness of the TiO₂ layer. The term "combination pigment" refers to more complex pigments that may contain a coated platelet such as titanium dioxide (the effect pigment or color variable pigment) coated mica to give a reflected color and an absorption pigment or dye which absorbs some portion of the visible spectrum. US4076551A relates to the use of carbon black in pigments where the pigment comprises a layer on the substrate of metal hydroxide or oxide or of bismuth oxychloride containing carbon black particles embedded therein. US5286291A describes a pigment containing carbon black, comprising a platelet-shaped substrate coated with a metal oxide layer containing carbon black, wherein the carbon black is fixed on the substrate by means of an anionic or cationic and a nonionic surfactant and an organosilane compound. EP1028146A1 discloses a colored interference pigment having a platelet-shaped layer as substrate, a coating of at least one metal oxide layer and, optionally, a further layer, at least two layers comprising or consisting of colorants.

### SUMMARY

The following summary provides a basic understanding of the embodiments of the disclosure. This summary is not an extensive overview of all contemplated aspects of the disclosure, and is not intended to identify all key or critical elements or to delineate the scope of any or all aspects of the disclosure. Its sole purpose is to present one or more aspects of the disclosure in a summary form as a prelude to the more detailed description of the invention that follows and the features described and particularly pointed out in the claims.

In one aspect of the present disclosure, a combination effect pigment comprises a platy substrate; an outer layer disposed above the platy substrate; and carbon black, wherein the carbon black is disposed above the substrate and is entrapped by the outer layer and wherein the carbon black is present in a form of hydrous oxide or hydroxide precipitate with a polyvalent cation selected from a group consisting of Al, Cr, Ti, Zn, Mg, Zr, Fe, Ce, and Sn is disclosed and is defined in claims 1 to 9.

In another aspect of the present disclosure, a paint, ink-jet, coatings, printing ink, plastic, cosmetic, glaze for ceramics, or glass comprising the combination effect pigment is disclosed and is defined in claim 10; a cosmetic comprising the combination effect pigment and is defined in claim 11; and a nail enamel comprising a nail enamel base and the combination effect pigment is disclosed and is defined in claims 12 to 14.

### BRIEF DESCRIPTION OF THE DRAWINGS

The above and other features of the present disclosure, their nature, and various advantages will become more apparent upon consideration of the following detailed description, taken in conjunction with the accompanying drawings, in which:
**Figures 1A** illustrates an exemplary layer arrangement for a combination effect pigment in accordance with an embodiment of the present disclosure;
**Figures 1B** illustrates another exemplary layer arrangement for a combination effect pigment in accordance with an embodiment of the present disclosure;
**Figures 1C** illustrates another exemplary layer arrangement for a combination effect pigment in accordance with an embodiment of the present disclosure;
**Figures ID** illustrates another exemplary layer arrangement for a combination effect pigment in accordance with an embodiment of the present disclosure;
**Figure 2** is a block diagram illustrating a method for producing a combination effect pigment in accordance with embodiments of the present disclosure; and
**Figure 3** is another block diagram illustrating a method for producing a combination effect pigment in accordance with embodiments of the present disclosure.

### DETAILED DESCRIPTION

The present disclosure is directed to combination pigments as disclosed in any one of claims 1 to 9. In particular, the present disclosure is directed to carbon black covered by or sandwiched between an additional layer. In some embodiments, the carbon black can be absorbed onto a porous substrate before the deposition of a layer on the platy substrate. The carbon black may be deposited, for example, by wet chemical deposition, chemical vapor deposition, or other processes. Carbon black deposition is followed by one or more overlayers. The one or more overlayers may be, for example, a high or low refractive index layer such as a metal oxide or SiO₂.

Other low refractive index substances, such as SiO₂, may be part of a combination pigment to influence the optical performance. One example is the creation of a color variable pigment, as described in U.S. Patent No. 6,875,264, which is hereby incorporated by reference herein in its entirety, and which discloses at least three layer effect pigments having, in sequence, a high refractive index layer, a low refractive index layer, and a high refractive index layer.

Traditional coating of carbon black on the surface of effect pigments led to a pigment which caused staining. This is especially problematic when such combination pigments are used in cosmetics. The carbon black treated effect pigment leaves a black residue on the skin or clothing. An objective of the present disclosure is to overlay the carbon black with at least one layer on a platy substrate. This minimizes the amount of free carbon particles on the effect pigment, thus reducing skin/clothing staining. A further objective is to maximize the color effect of the carbon black and the effect pigment.

Effect pigments are well known in the art and comprise a flake or platelet substrate that is coated with a reflecting layer. The reflection color is a function of optical thickness of the layer or layers on the substrate.

The term "combination effect pigment" refers to a combination of two types of color producing phenomena within the same pigment. For example, the combination effect pigment produces color as a function of optical thickness of a layered coating (e.g., TiO₂ coatings on mica) on a substrate, that acts as a reflecting layer which is a function of optical thickness, and includes an absorbing colorant on the same substrate (e.g., carbon black).

The term "optical layer" is well known in the art and refers to a coating or layer on a substrate where the coating or layer reflects color and the color is a function of the optical thickness of the coating, that is, the geometrical thickness times the refractive index of the coating. An optical thickness of 80 nm to 140 nm produce reflections which may be characterized as white, silvery, or pearly, and optical thicknesses of 190 nm or more produce colored reflections. Typically, the thickness of the optical coating or layer will range from 60 nm to 800 nm. The optical layer is most typically a metal oxide layer.

The term "at least one layer", as used herein, refers to one or more layers. The layer may be organic or inorganic. The "at least one layer" may be of high refractive index, that is greater than or equal to 1.65 or of low refractive index, that is of less than 1.65. The "at least one layer" may be a metal oxide selected from TiO₂, In₂O₃, ZrO₂, Fe₂O₃, Fe₃O₄, Cr₂O₃, CeO₂, ZnO, SnO₂, and mixtures thereof. The metal oxides TiO₂, Cr₂O₃, Fe₂O₃ and SnO₂ are most typical. Furthermore, the "at least one layer" may be a metal oxide, SiO₂, a metal such brass, bronze, silver or aluminum. In some embodiments, magnetite may be incorporated into one or more layers, or may be present as a standalone layer.

There may be multiple layers on the substrate overlayering the carbon black deposition and these may be formed from the as above metal oxide, SiO₂, a metal such as brass, bronze, silver and aluminum.

As used herein, the term "substrate" refers to platy inorganic or organic treated or untreated materials. For example, such platy materials may include aluminum oxide, platy glass, perlite, aluminum, natural mica, synthetic mica, bismuth oxychloride, platy iron oxide, platy graphite, platy silica, bronze, stainless steel, natural pearl, boron nitride, copper flake, copper alloy flake, zinc flake, zinc alloy flake, zinc oxide, enamel, china clay, porcelain, titanium oxide, platy titanium dioxide, titanium suboxide, kaolin, zeolites, and combinations thereof.

As used herein, the term "platy" (e.g., when referring to a "platy substrate") is well understood in the art. The term "platy" may be used interchangeably with flake, flake-like, plate-like, platelet and flaky.

A platy substrate has two dimensions (length and width) of similar magnitude and characteristically much greater than the third dimension (i.e., the thickness of the platy substrate). Platy substrates are useful for the application of the metal oxide coating and/or SiO₂ and deposition of the carbon black (e.g., via hydrous oxide or hydroxide precipitate of the polyvalent cation or co-deposition with, for example, a metal oxide or SiO₂ precursor).

In some embodiments, a diameter of a platy substrate (e.g., assuming a disc-like shape) may range from 0.1 to 350 microns, from 5 to 250 micrometers, or from 1 to 150 micrometers. In some embodiments, the diameter ranges from 5 to 50 or 100 micrometers.

In some embodiments, the substrate may be selected from a group consisting of iron oxide, synthetic mica, natural mica, basic lead carbonate, flaky barium sulfate, SiO₂, Al₂O₃, TiO₂, glass flakes, ZnO, ZrO₂, SnO₂, BiOCl, chromium oxide, BN, MgO flakes, Si₃N₄, graphite, aluminum, titanium, aluminum alloys, bronzes, iron, and perlite. In some embodiments, a platy substrate may be selected from a group consisting of synthetic mica, natural mica, SiO₂ flakes, Al₂O₃ flakes, TiO₂ flakes, Fe₂O₃ flakes, BiOCl, and glass flakes.

In some embodiments, the substrate may be treated or untreated. For example, the substrate may be treated with virtually any agent, such as silicones and coupling agents. In other embodiments, the substrate may be mechanically treated to smooth the surface, or plasma or radiation treated to activate the surface before deposition of the at least one layer or the carbon black.

In some embodiments, the substrate may also be a mixture of identical or different substrates, each having different particle sizes. The substrate mixture can include two, three or more different substrates, for example. In one embodiment, a mixture may include two or more of natural mica, synthetic mica, or glass flakes.

When applying the carbon black to the substrate directly or onto a SiO₂ layer, a porous substrate, for example, may be advantageous in some embodiments. A porous substrate or silica coated substrate can be treated with carbon black via incipient wetness impregnation. Incipient wetness is a process in which a solid support is impregnated with the maximum amount of solution that it can absorb without having any excess solution. After impregnation, the material is dried. In some embodiments, the solution includes a carbon black dispersion and a polyvalent cation.

In some embodiments, the carbon black can be any form of carbon black. In one embodiment, the carbon black comprises cosmetically approved Carbon Black No. 2.

In some embodiments, carbon black is deposited onto the substrate (or onto one or more intervening layers) via hydrous oxide or hydroxide precipitate with a polyvalent cation at a given pH. In some embodiments, carbon black is deposited onto the substrate (or onto one or more intervening layers) without a polyvalent cation. In some embodiments, carbon black is deposited in a form of carbon black particles. In some embodiments, carbon black is deposited as a standalone (single) layer disposed between the substrate and another layer (e.g., a layer comprising a different material than carbon black), and/or disposed between two layers (e.g., two layers comprising different material than carbon black). In some embodiments, carbon black is co-deposited with a layer, such that the carbon black is dispersed within the layer (e.g., a TiO₂ layer that includes carbon black dispersed throughout). In some embodiments, carbon black may be disposed between the substrate and another layer and/or between other pairs of adjacent layers. In some embodiments, carbon black may be deposited as a standalone (single layer) and may be partially dispersed within at least one of its adjacent layers. In some embodiments, various combinations of carbon black may be present as one or more carbon black layers along with one or more non-carbon black layers having carbon black dispersed therein.

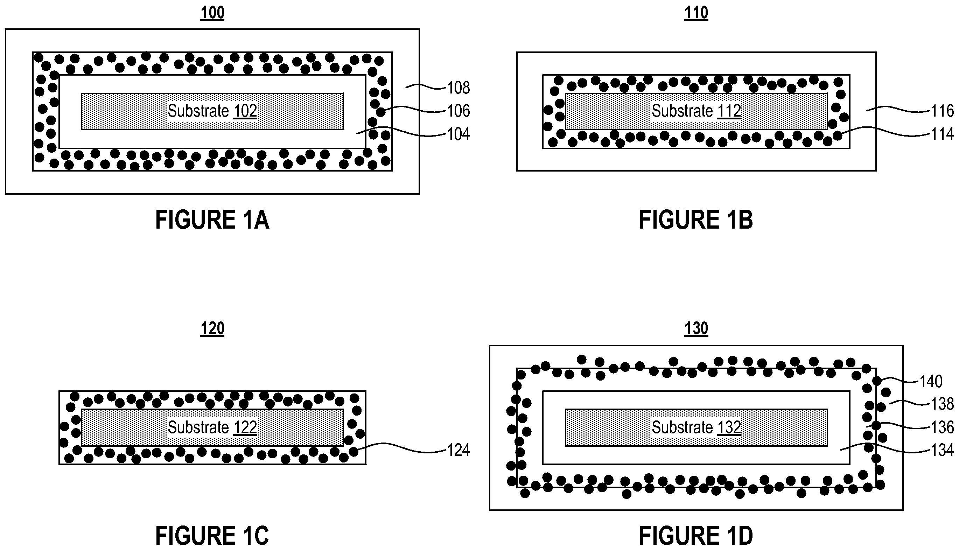

**Figures 1A-1D** illustrate different layer arrangements for a combination effect pigment in accordance with various embodiments of the present disclosure. The illustrations use generic terminology (e.g., layer, substrate, etc.) to denote the morphology, and it is to be understood that such generic terminology may correspond to any of the materials disclosed herein (e.g., a substrate may correspond to a platy substrate, an outer layer may comprises TiO₂, etc.). Moreover, it is noted that the drawings are not necessarily to scale. Other layer arrangements are also possible, and are described throughout the disclosure.

Pigment 100 includes a substrate 102 (e.g., a platy substrate), an intermediate layer 104 (e.g,. a metal oxide layer), an outer layer 108 (e.g., a metal oxide layer), and a carbon black layer 106 disposed between the intermediate layer 104 and the outer layer 108. In some embodiments, the carbon black layer 106 comprises a hydroxide precipitate of carbon black formed by a polyvalent cation. In some embodiments, the carbon black layer is a layer (e.g., a metal oxide layer) having carbon black particles dispersed therein.

Pigment 110 includes a substrate 112 (e.g., a platy substrate), an outer layer 116 (e.g., a metal oxide layer), and a carbon black layer 104 disposed between the substrate 112 and the outer layer 116.

Pigment 120 includes a substrate 122 (e.g., a platy substrate), and an outer layer 116 (e.g., a metal oxide layer) having carbon black (e.g., particles of carbon black) entrapped therein.

Pigment 130 includes a substrate 132 (e.g., a platy substrate), an inner layer 134 (e.g., a metal oxide layer), an intermediate layer 136 (e.g., a metal oxide layer), and an outer layer 138 (e.g., a metal oxide layer). Carbon black 140 may be dispersed at least partially throughout each of the intermediate layer 136 and the outer layer 140.

In some embodiments, the carbon black may first be dispersed in an aqueous medium in the presence of nonionic surfactants and/or anionic polymers before addition to a slurry containing the platy substrate. Without the dispersion of the carbon black, the carbon black may have a tendency to agglomerate and not deposit on the substrate when the hydrous or hydroxide precipitate is formed. Thus for example, the carbon black may be in a dispersed form when combined with the platy substrate before precipitation with the polyvalent cation onto the substrate.

Various loadings of carbon black may be utilized. As used herein, "wt. %" refers to an amount of loading of a material versus a starting substrate onto which the material is loaded. For example, 5 wt. % of carbon black would correspond to 5 g of carbon black loaded onto 100 g of platy substrate.

In some embodiments, carbon black may be present in the combination effect pigment in an amount, for example, up to 50.00 wt. %, up to 40.00 wt. %, up to 30.00 wt. %, from 0.01 wt. % to 50.00 wt. %, from 0.01 wt. % to 40.00 wt. %, from 0.01 wt. % to 30.00 wt. %, 0.01 wt. % to 25.00 wt. %, 0.01 wt. % to 20.00 wt. %, 0.01 wt. % to 15.00 wt. %, 0.01 wt. % to 10.00 wt. %, 0.01 wt. % to 5.00 wt. %, 0.01 wt. % to 3.00 wt. %, 0.01 wt. % to 1.00 wt. %, 0.01 wt. % to 0.80 wt. %, 0.01 wt. % to 0.50 wt. %, 0.01 wt. % to 0.30 wt. %, or 0.01 wt. % to 0.10 wt. %, based on a total weight of the uncoated platy substrate.

In some embodiments, carbon black may be present in the combination effect pigment in an amount from, for example, 0.10 wt. % to 50.00 wt. %, 0.10 wt. % to 40.00 wt. %, 0.10 wt. % to 30.00 wt. %, 0.10 wt. % to 25.00 wt. %, 0.10 wt. % to 20.00 wt. %, 0.10 wt. % to 15.00 wt. %, 0.10 wt. % to 10.00 wt. %, 0.10 wt. % to 5.00 wt. %, 0.10 wt. % to 3.00 wt. %, 0.10 wt. % to 1.00 wt. %, 0.10 wt. % to 0.80 wt. %, 0.10 wt. % to 0.50 wt. %, or 0.10 wt. % to 0.30 wt. %, based on a total weight of the uncoated platy substrate.

In some embodiments, carbon black may be present in the combination effect pigment in an amount from, for example, 1.00 wt. % to 50.00 wt. %, 1.00 wt. % to 40.00 wt. %, 1.00 wt. % to 30.00 wt. %, 1.00 wt. % to 25.00 wt. %, 1.00 wt. % to 20.00 wt. %, 1.00 wt. % to 15.00 wt. %, 1.00 wt. % to 10.00 wt. %, 1.00 wt. % to 5.00 wt. %, or 1.00 wt. % to 3.00 wt. %, based on a total weight of the uncoated platy substrate.

In some embodiments, carbon black may be present in the combination effect pigment in an amount from, for example, 10.00 wt. % to 50.00 wt. %, 10.00 wt. % to 40.00 wt. %, 10.00 wt. % to 30.00 wt. %, 10.00 wt. % to 25.00 wt. %, 10.00 wt. % to 20.00 wt. %, or 10.00 wt. % to 15.00 wt. %, based on a total weight of the uncoated platy substrate.

In some embodiments, carbon black may be present in the combination effect pigment in an amount from, for example, 10.00 wt. % to 50.00 wt. %, 10.00 wt. % to 40.00 wt. %, 10.00 wt. % to 30.00 wt. %, 10.00 to 25.00 wt. %, 10.00 to 20.00 wt. %, or 10.00 to about 15.00 wt. %, based on a total weight of the uncoated platy substrate.

In some embodiments, carbon black may be present in the combination effect pigment in an amount from, for example, 15.00 wt. % to 50.00 wt. %, 15.00 wt. % to 40.00 wt. %, 15.00 wt. % to 30.00 wt. %, 15.00 to 25.00 wt. %, or 15.00 to 20.00 wt. %, based on a total weight of the uncoated platy substrate.

Various loadings of carbon black may be utilized. In some embodiments, carbon black may be present in the combination effect pigment in an amount, for example, from 0.01 wt. % to 50.00 wt. %, from 0.01 wt. % to 40.00 wt. %, from 0.01 wt. % to 30.00 wt. %, 0.01 wt. % to 25.00 wt. %, 0.01 wt. % to 20.00 wt. %, 0.01 wt. % to 15.00 wt. %, 0.01 wt. % to 10.00 wt. %, 0.01 wt. % to 5.00 wt. %, 0.01 wt. % to 3.00 wt. %, 0.01 wt. % to 1.00 wt. %, 0.01 wt. % to 0.80 wt. %, 0.01 wt. % to 0.50 wt. %, 0.01 wt. % to 0.30 wt. %, or 0.01 wt. % to 0.10 wt. %, based on a total weight of the uncoated platy substrate.

In some embodiments, carbon black may be present in the combination effect pigment in an amount from, for example, 0.10 wt. % to 50.00 wt. %, 0.10 wt. % to 40.00 wt. %, 0.10 wt. % to 30.00 wt. %, 0.10 wt. % to 25.00 wt. %, 0.10 wt. % to 20.00 wt. %, 0.10 wt. % to 15.00 wt. %, 0.10 wt. % to 10.00 wt. %, 0.10 wt. % to 5.00 wt. %, 0.10 wt. % to 3.00 wt. %, 0.10 wt. % to 1.00 wt. %, 0.10 wt. % to 0.80 wt. %, 0.10 wt. % to 0.50 wt. %, or 0.10 wt. % to 0.30 wt. %, based on a total weight of the uncoated platy substrate.

In some embodiments, carbon black may be present in the combination effect pigment in an amount from, for example, 1.00 wt. % to 50.00 wt. %, 1.00 wt. % to 40.00 wt. %, 1.00 wt. % to 30.00 wt. %, 1.00 wt. % to 25.00 wt. %, 1.00 wt. % to 20.00 wt. %, 1.00 wt. % to 15.00 wt. %, 1.00 wt. % to 10.00 wt. %, 1.00 wt. % to 5.00 wt. %, or 1.00 wt. % to 3.00 wt. %, based on a total weight of the uncoated platy substrate.

In some embodiments, carbon black may be present in the combination effect pigment in an amount from, for example, 10.00 wt. % to 50.00 wt. %, 10.00 wt. % to 40.00 wt. %, 10.00 wt. % to 30.00 wt. %, 10.00 wt. % to 25.00 wt. %, 10.00 wt. % to 20.00 wt. %, or 10.00 wt. % to 15.00 wt. %, based on a total weight of the uncoated platy substrate.

In some embodiments, carbon black may be present in the combination effect pigment in an amount from, for example, 10.00 wt. % to 50.00 wt. %, 10.00 wt. % to 40.00 wt. %, 10.00 wt. % to 30.00 wt. %, 10.00 to 25.00 wt. %, 10.00 to 20.00 wt. %, or 10.00 to 15.00 wt. %, based on a total weight of the uncoated platy substrate.

In some embodiments, carbon black may be present in the combination effect pigment in an amount from, for example, 15.00 wt. % to 50.00 wt. %, 15.00 wt. % to 40.00 wt. %, 15.00 wt. % to 30.00 wt. %, 15.00 to 25.00 wt. %, or 15.00 to 20.00 wt. %, based on a total weight of the uncoated platy substrate.

In some embodiments, the polyvalent cation exists as a salt in an aqueous medium at the appropriate pH with a suitable anionic counterion, for example, selected from chloride, nitrate, sulfate, and combinations thereof. Any polyvalent cation which will form a precipitate under the given pH conditions may be used.

In some embodiments, the polyvalent cation is selected from Al, Cr, Ti, Zn, Mg, Zr, Fe and Sn. In some embodiments, the polyvalent cation is Al or Cr. In some embodiments, the polyvalent cation is selected from Al(III), Cr(III), Zn(II), Mg(II), Ti(IV), Zr(IV), Fe(II), Fe(III) and Sn(IV).

There are particular advantages using Cr(III), for example. When using CrCl₃·6H₂O, the precipitate formed with Cr(III) avoids agglomeration issues.

The formation of the hydrous or hydroxide precipitate works to deposit on the platy substrates a complex of a metal hydroxide or hydrous oxide which carries the carbon black dispersed particles with it, to produce the combination effect pigment with an adherent film on the platy substrates. This adherent film is sealed or overlaid with at least one layer (e.g., including an outer layer).

In some embodiments, a weight percent of polyvalent cation ranges from about 0.01 wt. % to 1 wt. %, 0.05 wt % to 0.9 wt %, or 0.1 wt % to 0.5 wt. %, based on the total weight of the uncoated platy substrate. In some embodiments, a weight percent of polyvalent cation ranges from 0.01 wt. % to 1 wt. %, 0.05 wt % to 0.9 wt %, or 0.1 wt % to 0.5 wt. %, based on the total weight of the uncoated platy substrate.

Furthermore the weight ratio of carbon black to the polyvalent cation ranges from 6:1 to 1:6, 5:1 to 1:5, 3:1 to 1:3, 2:1 to 1:2, or 1:1.5 to 1.5:1.

For example, the carbon black may be deposited on the platy substrate at a loading amount ranging from 0.1 wt. % to 0.5 wt. %, and the polyvalent cation amount may range from 0.1 wt. % to 0.5 wt. %, based on the total weight of the uncoated platy substrate. This would yield a weight ratio of carbon black to polyvalent cation ranging from 5:1 to 1:5.

As used herein, the term "carbon black precipitate" refers to a hydrous oxide or hydroxide precipitate formed in the presence of carbon black and a polyvalent cation at a given pH. The pH range for the deposition depends on the particular polyvalent cation being employed. For example, Al and Cr(III) will form a hydrous oxide or hydroxide precipitate between pH 4 and 9. The hydrous oxide or hydroxide precipitate may function as a binder that helps the carbon black to deposit directly onto the substrate or a layer disposed on the substrate.

In some embodiments, the carbon black or carbon black precipitate is deposited either directly onto the platy substrate or onto one of the layers on the platy substrate. The carbon black precipitate may be interposed as a carbon black layer between the substrate and the "at least one layer". The covering layer covers substantially the carbon precipitate layer. The covering layer may be a metal oxide, SiO₂, or a metal such as brass, bronze, silver, or aluminum.

### METHOD OF PREPARING COMBINATION EFFECT PIGMENT

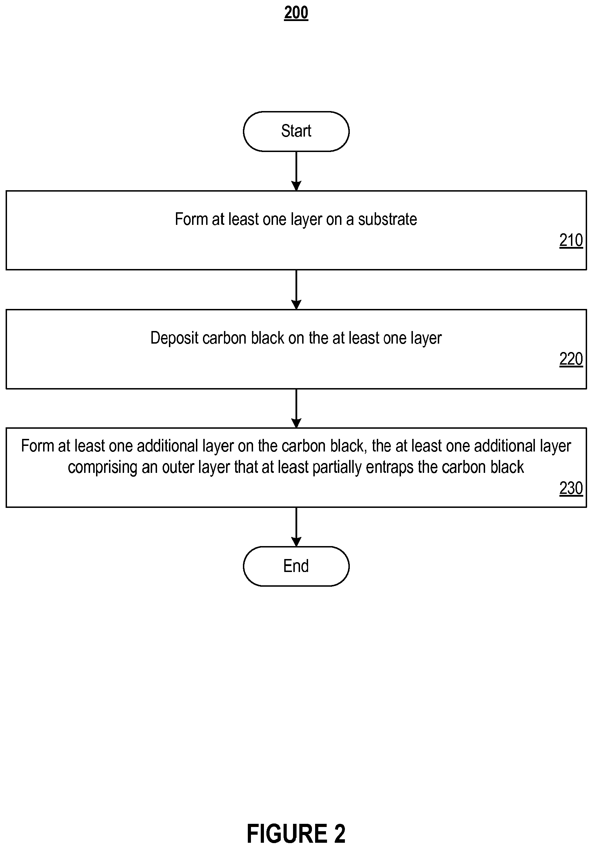

**Figure 2** is a block diagram illustrating a method 200 for producing a combination effect pigment in accordance with embodiments of the present disclosure.

At block 210, at least one layer is formed on a substrate. In some embodiments, the substrate is a platy substrate, as described herein. The at least one layer may include a single layer or multiple layers, with each being independently selected from, for example, a metal oxide, SiO₂, or a non-oxide metal (e.g., aluminum, silver, brass or bronze). In some embodiments, block 210 may be modified such that no layers are deposited onto the substrate, such that carbon black may be later deposited onto the substrate directly with no intervening layers in between.

In some embodiments, substrate may be in a suspension or slurry, and can be treated in order to deposit the at least one layer. Further the at least one layer may be present in various crystalline forms (e.g., a TiO₂ layer may be anatase or rutile). In some embodiments, the at least one layer may be deposited via chemical vapor deposition.

At block 220, carbon black is deposited on the at least one layer. In some embodiments, the carbon black is deposited via wet chemical deposition. In some embodiments, the carbon black is deposited via chemical vapor deposition. In other embodiments, other techniques may be utilized for depositing the carbon black, such as via incorporation of an organic moiety followed by a calcination step in an oxygen deprived atmosphere.

In some embodiments, wet chemical deposition may be performed by forming a slurry or suspension of carbon black, the substrate, and a polyvalent cation, with the slurry having a particular pH. In some embodiments, wet chemical deposition (and other types of deposition) may be performed without the use of a polyvalent cation.

In some embodiments, the suspension of the carbon black, substrate, and polyvalent cation (e.g., in salt form) is well-dispersed. The pH of the suspension may initially be maintained as acidic. The pH of the suspension may then be adjusted to form a precipitate of the desired polyvalent cation hydroxide or hydrous oxide, generally between 1 to 10. The carbon black may be deposited along with the hydroxide or hydrous oxide cation onto the at least one layer on the substrate (or onto the substrate itself if no layers are formed thereon) to form a smooth, uniform coating. The suspension can then be further treated to form at least one layer over the formed precipitate.

Any polyvalent cation which will form a precipitate under given pH conditions can be used. Such polyvalent cations may be used in a form of a solution of a soluble salt. In some embodiments, the polyvalent cations include one or more of Al(III), Cr(III), Zn(II), Mg(II), Ti(IV), Zr(IV), Fe(II), Fe(III), Ce(III) and Sn(IV). Suitable anions include chloride, nitrate, etc.

In embodiments that utilize a polyvalent cation, the pH range for wet chemical deposition may depend on the particular cation being used. The formation of the hydroxide or hydrous precipitate may be carried out by the addition of a soluble base, such as sodium hydroxide, potassium hydroxide, or ammonia solution where the desired pH of the precipitation is higher than the pH of the salt solution.

At block 230, at least one additional layer is formed on the carbon black, the at least one additional layers comprising an outer layer that at least partially entraps the carbon black. In some embodiments, the carbon black may be at least partially dispersed in the one or more additional layers. The combination effect pigment may then be dried after the outer layer is formed. The one or more additional layers may at least partially entrap (or completely entrap) the carbon black, which minimizes bleeding through of free carbon particles, thus reducing the staining of skin or clothing in certain applications of the combination effect pigment.

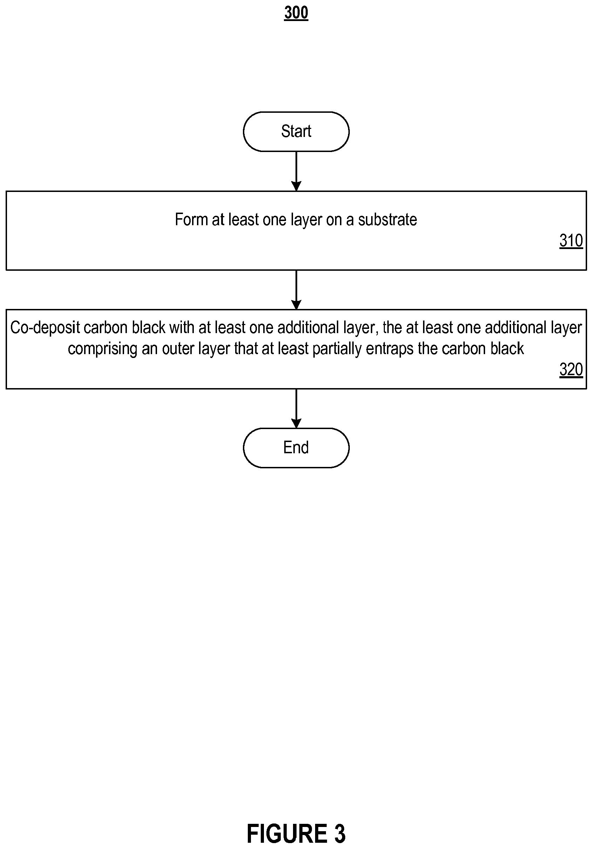

**Figure** 3 is a block diagram illustrating another method 300 for producing a combination effect pigment in accordance with embodiments of the present disclosure.

At block 310, at least one layer is formed on a substrate. In some embodiments, the substrate is a platy substrate, as described herein. Block 210 may be performed in a substantially similar manner as block 210 of **Figure 2****.** At block 320, carbon black is deposited on the at least one additional layer. For example, the carbon black, when co-deposited with another layer, may be dispersed as particles throughout the layer.

In each of methods 200 and 300, additional carbon black depositions and layer depositions may be performed as desired.

### ILLUSTRATIVE EXAMPLES

The following illustrative examples are set forth to assist in understanding the embodiments described herein and should not be construed as specifically limiting the embodiments described and claimed herein. Such variations, including the substitution of all equivalents now known or later developed, which would be within the purview of those skilled in the art, and changes in formulation or minor changes in experimental design, are to be considered to fall within the scope of the embodiments incorporated herein.

### Example 1: Carbon Black on Titania Coated Mica

A 13% aqueous slurry containing 100 grams of titania coated mica with gold interference color was heated to 78°C and stirred. The pH was adjusted to 3.0 using 20% w/w aq. HCl followed by the dumping of a carbon black dispersion (20% solids) for a 3.0 wt. % loading. The slurry was treated with 4.3% w/w aq. Al(NO₃)₃ for a 0.8 wt. % treatment. The slurry was then stirred for at least 10 minutes. Precipitation was further promoted by pumping of 3.5% w/w aq. NaOH at 1.5 mL/min and raising the pH to 8.0. The slurry was then allowed to stir for at least 30 minutes. Following the reaction described, the slurry was filtered, washed and dried at 120°C. The carbon black treated titania coated mica was compared to untreated titania coated mica, as a drawdown comprising 3% pigment in lacquer. The gold shade in the treated titanium coated mica was darkened. The bulk color of the treated titanium oxide coated mica was visibly gold, unlike the white bulk color observed for the untreated titanium oxide coated mica.

### Example 2: Carbon on Titania Coated Borosilicate Flake

A 33% aqueous slurry containing 200 g of titanium oxide coated borosilicate flakes with a pearl interference color was heated to 78°C and stirred. The pH was adjusted to 3.0 using 20% w/w aq. HCl. At pH 3.0, 20% w/w aq. carbon black dispersion is dumped for a 1.5 wt. % loading. The slurry was treated with 4.3% w/w aq. Al(NO₃)₃ for a 0.8 wt. % treatment followed by stirring for at least 10 minutes. Precipitation was further promoted by pumping of 3.5% w/w aq. NaOH at 1.5 mL/min and raising the pH to 8.0. The slurry was then allowed to stir for at least 30 minutes. Following the reaction described, the slurry was filtered, washed, and dried at 120°C. The treated product was compared to untreated titania coated borosilicate, as a drawdown comprising 12% pigment in a lacquer. An enhanced darkened pearl shade was observed in the treated product compared to untreated starting titanium oxide coated borosilicate flakes. Further, the bulk color of the carbon treated flakes was clearly a silver pearl compared to the white bulk color observed in the untreated flakes.

### Example 3: Carbon Black on the first TiO₂ Layer Using Al(NO₃)₃

A 33% aqueous slurry containing 832 g of glass flakes (5 micrometer thickness and 100 micrometer mean diameter) was heated to 80°C and stirred. The pH was adjusted to 1.35 with 28% aq. w/w HCl, then 47g of 20% w/w SnCl₄ solution was pumped in at a pH of 1.35 at 1.94 mL/min. A 40% w/w aq. TiCl₄ solution was then pumped at 1.5 ml/min to match a pearl shade. The pH was adjusted to 3.0 for the addition of carbon black. The 20% carbon black dispersion was dumped in for a 0.2 wt. % loading. The carbon black dump is followed by the addition of 4.3% w/w aq. Al(NO₃)₃ for a 0.1 wt. % treatment. Precipitation was further promoted by pumping 3.5% w/w NaOH and raising the pH to 8.0 at 1.5mL/min. The slurry was then allowed to stir for at least 30 minutes. At pH 8.0, 458.7 g of 20% w/w aq. Na₂SiO₃·5H₂O aqueous solution are pumped at 4.0g/min using 28% w/w HCl to control the pH. Following the Na₂SiO₃·5H₂O addition, pH is adjusted to 1.4 by pumping 28% w/w HCl at 2.9 g/min for SnCl₄ addition. Then 47 g of 20% w/w SnCl₄ solution was added at 2.4 g/min using 35% w/w aq. NaOH to control the pH, followed by 164 g of 40% w/w TiCl₄ using 35% w/w NaOH to control the pH at 1.4. Following the reaction described, the slurry was filtered, washed, and heat-treated at 400°C. The carbon black treated flakes were compared to flakes made without the carbon black treatment, as a drawdown comprising 12% pigment in SLF2 lacquer. A much darker pearl shade was observed in the treated flakes compared to flakes prepared without the carbon black treatment. The bulk color of the carbon black containing flakes was a dark pearl in contrast to the colorless bulk of the flakes not treated with carbon black. It is noted that using this same procedure, carbon black can be incorporated on any of the layers of the combination effect pigment described.

### Example 4: Carbon Black on the First TiO₂ Layer Using CrCl₃·5H₂O

A 33% aqueous slurry containing 832 g of glass flakes (5 micrometer thickness and 100 micrometer mean diameter) was heated to 80°C and stirred. The pH was adjusted to 1.35 with 28% w/w aq. HCl, then 47 g of a 20% w/w aq. SnCl₄ solution was pumped in at a pH of 1.35 at 1.94 mL/min. A 40% w/w aq. TiCl₄ solution was pumped at 2.4 g/min to match a pearl shade. The pH was raised to 3.0 for the addition of carbon black. The 20% carbon black dispersion was dumped in for a 0.3 wt. % loading. The pH was raised to 6.8 using 3.5% w/w aq. NaOH, then 1.5% w/w aq. CrCl₃·5H₂O was pumped in for a 0.5wt. % treatment using 3.5% w/w NaOH to maintain pH. Precipitation was further promoted by pumping of 3.5% w/w NaOH and raising the pH to 7.5. The slurry was then allowed to stir for at least 20 minutes. At pH 8.0, 458.7 g of 20% w/w aq. Na₂SiO₃·5H₂O aqueous solution was pumped at 4.0 g/min using 28% w/w aq. HCl to control the pH. Following the Na₂SiO₃·5H₂O addition, pH was adjusted to 1.4 for SnCl₄ addition by pumping 28% w/w HCl at 2.9 g/min, followed by 47 g of 20% w/w SnCl₄ solution added at 1.94 mL/min using 35% w/w NaOH to control the pH. Next, 164 g of 40% w/w TiCl₄ were then pumped at 2.4 g/min using 35% w/w NaOH to control the pH. Following the reaction described, the slurry was filtered, washed, and heat-treated at 400°C. The carbon treated flakes were compared to the same flakes made above but without carbon black treatment, as a drawdown comprising 12% pigment in SLF2 lacquer. A much darker pearl shade can be observed for the carbon black containing pigment while keeping the sparkle effect observed in untreated flakes. The bulk color of the carbon black containing pigment is also representative of the color observed in the drawdown as opposed to the white bulk color observed for a same pigment without the carbon black treatment. It is noted that using this same procedure, carbon black can be incorporated on any of the layers of the combination effect pigment described.

### Example 5: Carbon Black in the Second TiO₂ Layer of a Violet Effect Pigment Using CrCl₃·5H₂O

A 13.4% aqueous slurry containing 258 g of glass flakes (1 micrometer thickness and 80 micrometers mean diameter)was heated to 82°C and stirred. The pH is adjusted to 1.3 with 1:1 v/v HCl solution, then 42 g of a 20% w/w aq. SnCl₄ solution was pumped in at a pH of 1.3 at 2.2g/min. A 40% w/w aq. TiCl₄ solution was pumped at 2.4 g/min to match a pearl shade. The pH was then increased to 7.8, then 1200 g of 20% aq. w/w Na₂SiO₃·5H₂O was pumped at 4.0g/min using 28% w/w HCl to control the pH. Following the Na₂SiO₃·5H₂O addition, pH was adjusted to 1.7 then 8.0 g of 20% w/w SnCl₄ solution were dumped into the slurry. Next, 90 g of 40% w/w TiCl₄ was pumped in using 35% w/w NaOH to control the pH. After, the pH was raised to 3.0 for the addition of darbon black. The 20% carbon black dispersion was dumped into the slurry for a 0.4 wt. % loading. The pH was then raised to 6.8 using 3.5% w/w NaOH followed by 1.5% w/w aq. CrCl₃·5H₂O that was pumped in for a 0.6 wt. % treatment using 3.5% w/w NaOH to maintain pH. Precipitation was further promoted by pumping of 3.5% w/w NaOH and raising the pH to 7.5. After a 20 minutes stir, the pH of the slurry was lowered to 1.35. An additional 30 g of 40% w/w TiCl₄ were added. Following the reaction described, the slurry was filtered, washed, and heat-treated at 400°C. The treated flakes were compared to the effect pigment prepared in the same way except not treated with carbon black, as a drawdown comprising 6% pigment in DL101 lacquer. The color travel observed in the carbon black treated pigment was in line with that observed in the untreated violet: red to violet. The bulk color of the treated pigment is of a violet shade. It is noted that using this same procedure, carbon black can be incorporated on any of the layers of the combination effect pigment described.

### Example 6: Carbon Black in the Second TiO₂ Layer Using FeCl₃

A 13.4% aqueous slurry containing 258 g of glass flakes (1 micrometer thickness and 80 micrometer mean diameter) was heated to 82°C and stirred. The pH was adjusted to 1.3 with 1:1 v/v HCl solution, then 42 g of a 20% w/w aq. SnCl₄ solution was pumped in at a pH of 1.3 at 2.2 g/min. Next, a 40% w/w aq. TiCl₄ solution is pumped to match a pearl shade. The pH was then increased to 7.8 and 1200 g of 20% w/w Na₂SiO₃·5H₂O aqueous solution was pumped at 4.0 g/min using 28% w/w aq. HCl to control the pH. Following the Na₂SiO₃·5H₂O addition, the pH was adjusted to 1.7 for SnCl₄ addition by pumping 28 %w/w HCl at 2.9 g/min. After 8.0 g of 20% w/w SnCl₄ solution were dumped into the slurry, 97.5 g of 40% w/wTiCl₄ were then pumped using 35% w/w NaOH to control the pH. The pH was then raised to 3.0 for the addition of carbon black. The 20% carbon black dispersion was dumped into the slurry for a 0.4 wt. % loading. Next, 2.29% w/w aq. FeCl₃ was pumped in for a 0.6 wt. % treatment using 3.5% w/w NaOH to maintain pH. The pH was then lowered to 1.35, and an additional 32.5 g of 40% w/w TiCl₄ was added. Following the reaction described, the slurry was filtered, washed, and heat-treated at 400°C. The carbon black treated flakes were compared to the same flakes but not coated with carbon black, as a drawdown comprised 6% pigment in DL101 lacquer. The color travel observed in carbon black treated pigment was in line with that observed in same flakes not treated with carbon black, blue to violet. The bulk color of the carbon black treated flakes was violet.

### Example 7: Carbon Black in Silica Layer

A 33% aqueous slurry containing 832 g of glass flakes (5 micrometer thickness and 100 micrometers mean diameter) was heated to 80°C and stirred. The pH was adjusted to 1.35 with 1:1 v/v HCl solution, then 47 g of a 20% w/w aq. SnCl₄ solution was pumped in at a pH of 1.3 at 2.2 g/min. Next, a 40% w/w aq. TiCl₄ solution was pumped at 2.4 g/min to match a pearl shade. The pH was then increased to 7.8, and 1075 g of 20% w/w Na₂SiO₃·5H₂O aqueous solution was pumped at 4.0 g/min using 28% w/w aq. HCl to control the pH. The slurry was filtered, washed, and dried at 120°C to make the silica intermediate. Then 350 g of the silica intermediate were impregnated with 35 g of 2% w/w aq. carbon black dispersion solution via incipient wetness impregnation. The substrate was dried at 120°C followed by the impregnation of 35 g of 1.5% w/w aq. Cr(NO₃)₃·9H₂O solution. The substrate was dried at 120°C. At this point the bulk color of the pigment was a dark intense gold. Thereafter, the substrate was reslurried in 700 mL water and heated to 82°C and stirred. The pH was adjusted to 1.6, then 2.7 g of 20% w/w SnCl₄ was dumped into the slurry, followed by at least 22 minutes stir. The pH was then adjusted to 1.35 for TiCl₄ addition by pumping 28% w/w HCl. Next, 67.6 g of 40% w/w TiCl₄ was pumped in at 2.4 g/min to match Reflecks™ MultiDimensions Transforming Teal. Following the reaction described, the slurry was filtered, washed, and heat-treated at 400°C. A weak color travel from light red to a slight green shade was observed in the bulk color. A similar effect was observed in a drawdown comprising 6% pigment in DL101 lacquer.

### Example 8: Carbon Black in the Second TiO₂ Layer Using CrCl₃·5H₂O

A 13.4% aqueous slurry containing 258 g of glass flakes (1 micrometer thickness and 80 micrometers mean diameter) was heated to 82°C and stirred. The pH was adjusted to 1.3 with 1:1 v/v HCl solution, then 42 g of a 20% w/w aq. SnCl₄ solution was pumped in at a pH of 1.3 at 2.2 g/min. A 40% w/w aq. TiCl₄ solution was pumped at 2.4 g/min to match a pearl shade. The pH was then increased to 7.8, and 1075 g of 20% aq. w/w Na₂SiO₃·5H₂O was pumped in at 4.0 g/min using 28% w/w HCl to control the pH. Following the Na₂SiO₃·5H₂O addition, pH was adjusted to 1.7 for SnCl₄ addition. After 8.0 g of 20% w/w SnCl₄ addition was dumped into the slurry, 90 g of 40% w/w TiCl₄ was then pumped in using 35% w/w NaOH to control the pH. The pH was then raised to 3.0 for the addition of carbon black. The 20% carbon black dispersion was dumped into the slurry for a 0.4 wt. % loading. The pH was raised to 6.8 using 3.5% w/w NaOH, followed by 1.5% w/w aq. CrCl₃·5H₂O that was pumped in for a 0.6 wt. % treatment using 3.5% w/w NaOH to maintain pH. Precipitation was further promoted by pumping of 3.5% w/w NaOH and raising the pH to 7.5. After a 20 minutes stir, the pH of the slurry was lowered to 1.35. An additional 50 g of 40% w/w TiCl₄ was then added. Following the reaction described, the slurry was filtered, washed, and heat-treated at 400°C. The carbon black treated pigment was compared to pigment without the carbon black treatment, as a drawdown comprising 6% pigment in DL101 lacquer. The color travel observed in carbon black treated pigment was in line with that observed in the same pigment without the carbon black treatment: red to violet. The bulk color of untreated pigment is of a violet shade. It is noted that the use of CrCl₃·5H₂O as the polyvalent cation demonstrated noticeable improvements over Al(NO₃)₃. Specifically, no agglomeration issues were observed with the use of CrCl₃·5H₂O, which is characteristic of high loadings of Al(NO₃)₃.

### Example 9: Polyvalent Cation-Free Combination Effect Pigment

A 10% aqueous slurry containing 230 g of glass flakes was heated to 80°C and stirred. The pH was adjusted to 1.3 with 28% w/w aq. HCl, then 43 g of a 20% w/w aq. SnCl₄ solution was pumped in at a pH of 1.3 at 2 mL/min. A 25% w/w aq. TiOCl₂ solution was subsequently added at 2.4 g/min. Once a given amount of TiCl₄ had been added, a 10% carbon black dispersion was simultaneously added to the reaction in order to achieve a 1 wt. % coating. The carbon black coating on the substrate was facilitated by the deposition of TiO₂ onto the substrate. Once the desired pigment shade had been achieved, the reaction was stopped. Following the reaction described, the slurry was filtered, washed and calcined at 400°C. The carbon black treated coated glass flakes was compared to untreated titania coated glass flakes as a drawdown comprising 6% pigment in lacquer. The bulk color of the carbon black treated flakes had a darkened bulk color in comparison to untreated flakes.

### Example 10: Cosmetic Applications

Preliminary tests revealed that incorporating carbon black into an effect pigment with an overlayer is advantageous versus a physical blend of the carbon black and an effect pigment. Skin staining is significantly diminished. Further, incorporating the carbon within a layer is expected to improve upon the external carbon attachment to an effect material.

### ADDITIONAL ILLUSTRATIVE EXAMPLES

The following additional illustrative examples are set forth to assist in understanding the embodiments described herein. Such variations, including the substitution of all equivalents now known or later developed, which would be within the purview of those skilled in the art, and changes in formulation or minor changes in experimental design, are to be considered to fall within the scope of the embodiments incorporated herein.

### Example A1: Carbon Black in the Second TiO₂ Layer Using AlCl₃ in a Blue Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution. 42 g of a 20% w/w aq. SnCl₄ solution was pumped in at a rate of 2.2 g/min. A 25% w/w aq. TiOCl₂ solution was pumped in at 2.4g/min to match a pearl shade. The pH was then increased to 7.8, and 1285 g of 20% aq. w/w Na₂SiO₃·5H₂O was pumped in at 8.0 g/min using 28% w/w HCl to control the pH. Following the Na₂SiO₃·5H₂O addition, pH was adjusted to 1.7 for SnCl₄ addition. After 8.0 g of 20% w/w SnCl₄ addition was dumped into the slurry, 50 g of 25% w/w TiOCl₂ was then pumped in using 35% w/w NaOH to control the pH. At pH 1.3-1.4, carbon black was added. The 25% carbon black dispersion was dumped into the slurry for a 0.4 wt. % loading. The pH was raised to 6.8 using 35% w/w NaOH, followed by 5% w/w aq. AlCl₃ that was pumped in for a 0.6 wt. % treatment using 3.5% w/w NaOH to maintain pH. Precipitation was further promoted by pumping of 3.5% w/w NaOH and raising the pH to 7.5. After stirring for 60 minutes, the pH of the slurry was lowered to 1.40. An additional 45 g of 25% w/w TiOCl₂ was then added. Following the reaction described, the slurry was filtered, washed, and heat-treated at 400°C. The carbon black treated pigment was compared to pigment without the carbon black treatment, as a drawdown comprising 6% pigment in DL1000 lacquer. The color travel observed in carbon black treated pigment was in line with that observed in the same pigment without the carbon black treatment, blue to purple. The bulk color of the treated carbon black powder is a bluish-purple. The bulk color of untreated pigment is of a white shade. The resulting layer structure is platy substrate/TiO₂/SiO₂/TiO₂/carbon black/TiO₂.

### Example A2: Carbon Black in the Second TiO₂ Layer Using AlCl₃ in a Blue Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution, then 42 g of a 20% w/w aq. SnCl₄ solution were pumped in at a rate of 2.2 g/min. A 25% w/w aq. TiOCl₂ solution was pumped at 2.4 g/min to match a pearl shade. The pH was then increased to 7.8, and 1285 g of 20% aq. w/w Na₂SiO₃·5H₂O were pumped at 8.0 g/min using 28% w/w HCl to control the pH. Following the Na₂SiO₃·5H₂O addition, pH was adjusted to 1.7 for SnCl₄ addition. After 8.0 g of 20% w/w SnCl₄ addition was dumped into the slurry, 50 g of 25% w/w TiOCl₂ was then pumped in using 35% w/w NaOH to control the pH. At pH 1.3-1.4, carbon black was added. The 25% carbon black dispersion was dumped into the slurry for a 0.6 wt. % coating. The pH was raised to 6.8 using 35% w/w NaOH, followed by 5% w/w aq. AlCl₃ that was pumped in for a 0.8 wt. % treatment using 3.5% w/w NaOH to maintain pH. Precipitation was further promoted by pumping of 3.5% w/w NaOH and raising the pH to 7.5. After stirring for 60 minutes, the pH of the slurry was lowered to 1.40. An additional 45 g of 25% w/w TiOCl₂ was then added. Following the reaction described, the slurry was filtered, washed, and heat-treated at 400°C. The carbon black treated pigment was compared to pigment without the carbon black treatment, as a drawdown comprising 6% pigment in DL1000 lacquer. The color travel observed in the carbon black treated pigment was in line with that observed in the same pigment without the carbon black treatment, blue to purple. The bulk color of the treated carbon black powder is a bluish-purple. The bulk color of untreated pigment is of a white shade.

### Example A3: Carbon Black in the Second TiO₂ Layer Using AlCl₃ in a Blue Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution, then 42 g of a 20% w/w aq. SnCl₄ solution was pumped in at a rate of 2.2 g/min. A 25% w/w aq. TiOCl₂ solution was pumped in at 2.4 g/min to match a pearl shade. The pH was then increased to 7.8, and 1285 g of 20% aq. w/w Na₂SiO₃·5H₂O was pumped in at 8.0 g/min using 28% w/w HCl to control the pH. Following the Na₂SiO₃·5H₂O addition, pH was adjusted to 1.7 for SnCl₄ addition. After 8.0 g of 20% w/w SnCl₄ addition was dumped into the slurry, 50 g of 25% w/w TiOCl₂ was then pumped in using 35% w/w NaOH to control the pH. At pH 1.3-1.4, carbon black was added. The 25% carbon black dispersion was dumped into the slurry for a 1.0 wt. % coating. The pH was raised to 6.8 using 3.5% w/w NaOH, followed by 5% w/w aq. AlCl₃ that was pumped in for a 0.6 wt. % treatment using 3.5% w/w NaOH to maintain pH. Precipitation was further promoted by pumping of 3.5% w/w NaOH and raising the pH to 7.5. After stirring for 60 minutes, the pH of the slurry was lowered to 1.40. An additional 45 g of 25% w/w TiOCl₂ was then added. Following the reaction described, the slurry was filtered, washed, and heat-treated at 400°C. The carbon black treated pigment was compared to pigment without the carbon black treatment, as a drawdown comprising 6% pigment in DL1000 lacquer. The color travel observed in the carbon black treated pigment was in line with that observed in the same pigment without the carbon black treatment: blue to purple. The bulk color of the treated carbon black powder is a bluish-purple. The bulk color of untreated pigment is of a white shade.

### Example A4: Carbon Black in the Second TiO₂ Layer Using AlCl₃ in a Red Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution, then 42 g of a 20% w/w aq. SnCl₄ solution was pumped in at a rate of 2.2 g/min. A 25% w/w aq. TiOCl₂ solution was pumped in at 2.4 g/min to match a pearl shade. The pH was then increased to 7.8, and approximately 2090 g of 20% aq. w/w Na₂SiO₃·5H₂O was pumped in at 8.0 g/min using 28% w/w HCl to control the pH. Following the Na₂SiO₃·5H₂O addition, pH was adjusted to 1.7 for SnCl₄ addition. After 8.0 g of 20% w/w SnCl₄ addition was dumped into the slurry, 50 g of 25% w/w TiOCl₂ were then pumped using 35% w/w NaOH to control the pH. At pH 1.3-1.4, carbon black was added. The 25% carbon black dispersion was dumped into the slurry for a 0.4 wt. % coating. The pH was raised to 6.8 using 3.5% w/w NaOH, followed by 5% w/w aq. AlCl₃ that was pumped in for a 0.6 wt. % treatment using 3.5% w/w NaOH to maintain pH. Precipitation was further promoted by pumping of 3.5% w/w NaOH and raising the pH to 7.5. After stirring for 60 minutes, the pH of the slurry was lowered to 1.40. An additional 30 g of 25% w/w TiOCl₂ was then added. Following the reaction described, the slurry was filtered, washed, and heat-treated at 400°C. The carbon black treated pigment was compared to pigment without the carbon black treatment, as a drawdown comprising 6% pigment in DL1000 lacquer. The color travel observed in the carbon black treated pigment was in line with that observed in the same pigment without the carbon black treatment, red to pale green. The bulk color of the treated carbon black powder is a dark red. The bulk color of untreated pigment is of a white shade.

### Example A5: Carbon Black in the Second TiO₂ Layer Using AlCl3 in a Red Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution, then 42 g of a 20% w/w aq. SnCl₄ solution was pumped in at a rate of 2.2 g/min. A 25% w/w aq. TiOCl₂ solution was pumped in at 2.4 g/min to match a pearl shade. The pH was then increased to 7.8, and approximately 2090 g of 20% aq. w/w Na₂SiO₃·5H₂O was pumped in at 8.0 g/min using 28% w/w HCl to control the pH. Following the Na₂SiO₃·5H₂O addition, pH was adjusted to 1.7 for SnCl₄ addition. After 8.0 g of 20% w/w SnCl₄ addition was dumped into the slurry, 50 g of 25% w/w TiOCl₂ was then pumped in using 35% w/w NaOH to control the pH. At pH 1.3-1.4, carbon black was added. The 25% carbon black dispersion was dumped into the slurry for a 1.0 wt. % coating. The pH was raised to 6.8 using 3.5% w/w NaOH, followed by 5% w/w aq. AlCl₃ that was pumped in for a 0.6 wt. % treatment using 3.5% w/w NaOH to maintain pH. Precipitation was further promoted by pumping of 3.5% w/w NaOH and raising the pH to 7.5. After stirring for 60 minutes, the pH of the slurry was lowered to 1.40. An additional 30 g of 25% w/w TiOCl₂ was then added. Following the reaction described, the slurry was filtered, washed, and heat-treated at 400°C. The carbon black treated pigment was compared to pigment without the carbon black treatment, as a drawdown comprising 6% pigment in DL1000 lacquer. The color travel observed in the carbon black treated pigment was in line with that observed in the same pigment without the carbon black treatment: red to pale green. The bulk color of the treated carbon black powder is a dark red. The bulk color of untreated pigment is of a white shade.

### Example A6: Carbon Black Between the First TiO₂ layer and the Silica layer Using AlCl₃ in a Blue Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution, then 42 g of a 20% w/w aq. SnCl₄ solution was pumped in at a rate of 2.2 g/min. A 25% w/w aq. TiOCl₂ solution was pumped at 2.4 g/min to match a pearl shade. The pH was adjusted to 3.0 for the addition of carbon black. The 25% carbon black dispersion was dumped in for a 0.4 wt. % coating. The carbon black dump was followed by the addition of 5% w/w aq. AlCl₃ for a 0.6 wt. % treatment. Precipitation was further promoted by pumping 3.5% w/w NaOH and raising the pH to 6.8 at 2 g/min. The slurry was then allowed to stir for at least 30 minutes. At pH 7.8, 1265 g of 20% w/w aq. Na₂SiO₃·5H₂O aqueous solution was pumped in at 8.0 g/min using 28% w/w HCl to control the pH. Following the Na₂SiO₃·5H₂O addition, pH was adjusted to 1.3 by pumping 28% w/w HCl at 2.9 g/min for SnCl₄ addition. Then 8 g of 20% w/w SnCl₄ solution was added by a dump process. 25% w/w TiOCl₂ was added at a rate of 2.4 g/min using 35% w/w NaOH to control the pH at 1.3. Following the reaction described, the slurry was filtered, washed, and heat-treated at 400°C. The carbon black treated flakes were compared to flakes made without the carbon black treatment, as a drawdown comprised of 0.6% pigment in DL1000 lacquer. The color travel observed in the carbon black treated pigment was in line with that observed in the same pigment without the carbon black treatment: blue to purple. The bulk color of the treated carbon black powder is a bluish-purple. The bulk color of untreated pigment is of a white shade.

### Example A7: Carbon Black in the Silica Layer Using AlCl3 in a Blue Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution, then 42 g of a 20% w/w aq. SnCl₄ solution were pumped in at a rate of 2.2 g/min. A 25% w/w aq. TiOCl₂ solution was pumped in at 2.4 g/min to match a pearl shade. The pH was then increased to 7.8, and 760 g of 20% w/w Na₂SiO₃·5H₂O aqueous solution was pumped in at 8.0 g/min using 28% w/w aq. HCl to control the pH. The pH was adjusted to 3.0 for the addition of carbon black. The 25% carbon black dispersion was dumped in for a 0.4wt. % coating. The carbon black dump was followed by the addition of 5% w/w aq. AlCl₃ for a 0.6 wt. % treatment. Precipitation was further promoted by pumping 3.5% w/w NaOH and raising the pH to 6.8 at 2 g/min. The slurry was then stirred for at least 30 minutes. 20% w/w Na₂SiO₃·5H₂O aqueous solution was pumped at 8.0 g/min using 28% w/w aq. HCl to control the pH to complete the silica layer. At this point, the bulk color of the slurry was a dark intense gold. The pH was adjusted to 1.7 and 8 g of 20% w/w SnCl₄ was dumped into the slurry, followed by stirring for 22 minutes. The pH was then adjusted to 1.30. 25% w/w TiOC12 was added at a rate of 2.4 g/min using 35% w/w NaOH to control the pH at 1.3. Addition continued until the desired blue shade was reached. Following the reaction described, the slurry was filtered, washed, and heat-treated at 400°C. A weak color travel from blue to purple shade was observed in the bulk color. A similar effect was observed in a drawdown comprising 6% pigment in DL1000 lacquer.

### Example A8: Carbon Black between a Silica Layer and the 2nd TiO₂ Layer Using AlCl3 in a Blue Pigment

A 15.5% aqueous slurry containing 258 g of glass flakes (1 micrometer thickness and 80 micrometers mean diameter) was heated to 82°C and stirred. The pH was adjusted to 1.3 with 28% HCl solution, then 42 g of a 20% w/w aq. SnCl₄ solution was pumped in at a rate of 2. 2g/min. A 25% w/w aq. TiOCl₂ solution was pumped at 2.4 g/min to match a pearl shade. The pH was then increased to 7.8, and 1285 g of 20% aq. w/w Na₂SiO₃·5H₂O were pumped in at 8.0 g/min using 28% w/w HCl to control the pH. Following the Na₂SiO₃·5H₂O addition, the pH of the slurry was adjusted to 3.0. The 25% carbon black dispersion was dumped into the slurry for a 0.4 wt. % coating. The pH was raised to 6.8 using 3.5% w/w NaOH, followed by 5% w/w aq. AlCl₃ that was pumped in for a 0.6 wt. % treatment using 3.5% w/w NaOH to maintain pH. Precipitation was further promoted by pumping of 3.5% w/w NaOH and raising the pH to 7.5. After a 20 minutes stir, the pH was adjusted to 1.7 for SnCl₄ addition. After 8.0g of 20% w/w SnCl₄ was dumped into the slurry, the slurry was stirred for 22 minutes. 25% w/w TiOCl₂ was then pumped in using 35% w/w NaOH to control the pH. TiOCl₂ addition continued until the desired blue shade was reached. Following the reaction described, the slurry was filtered, washed, and heat-treated at 400°C. The carbon black treated pigment was compared to pigment without the carbon black treatment, as a drawdown comprising 6% pigment in DL1000 lacquer. The color travel observed in the carbon black treated pigment was in line with that observed in the same pigment without the carbon black treatment: blue to purple. The bulk color of the treated carbon black powder is a bluish-purple. The bulk color of untreated pigment is of a white shade. The resulting layer structure is platy substrate/TiO₂/SiO₂/carbon black/TiO₂.

### EXEMPLARY APPLICATIONS OF EMBODIMENTS

The combination effect pigments according to the present disclosure can be used for all customary purposes, for example, for coloring polymers in a bulk material, coatings (including effect finishes and those for the automotive sector), printing inks (including offset printing, intaglio printing, gravure, bronzing, and flexographic printing), applications in cosmetics, in ink-jet-printing, for dyeing textiles, as well as laser marking of papers and plastics. Such applications are known from reference works, for example "Industrielle Organische Pigmente" (W. Herbst and K. Hunger, VCH Verlagsgesellschaft mbH, Weinheim - New York, 2nd, completely revised edition, 1995).

A paint, coating, printing ink, plastic, cosmetic formulation, laser marking, pigment composition or dry preparation, and a cosmetic formulation are example embodiments in which a combination effect pigment of the present disclosure may be incorporated.

In one embodiment, the combination effect pigment is part of a cosmetic composition. The form of the cosmetic composition can be any form normally used for cosmetics such as a cream, emulsion, foam, gel, lotion, milk, mousse, ointment, paste, powder, spray, or suspension. The cosmetic composition can be any colored cosmetic used on the skin, hair, eyes, or lips, such as concealing sticks, foundation, stage make-up, mascara (cake or cream), eye shadow (liquid, pomade, powder, stick, pressed, or cream), hair color, lipsticks, lip gloss, kohl pencils, eye liners, blushers, eyebrow pencils, and cream powders. Other exemplary cosmetic compositions include, but are not limited to, nail enamel, skin glosser stick, hair sprays, face powder, leg-makeup, insect repellent lotion, nail enamel remover, nail enamel base, perfume lotion, and shampoos of all types (gel or liquid). In addition, the claimed compositions can be used in shaving cream (concentrate for aerosol, brushless, lathering), hair groom, cologne stick, cologne, cologne emollient, bubble bath, body lotion (moisturizing, cleansing, analgesic, astringent), after shave lotion, after bath milk and sunscreen lotion. For a review of cosmetic applications, see Cosmetics: Science and Technology, 2nd Ed., Eds: M. S. Balsam and Edward Sagarin, Wiley-Interscience (1972) and deNavarre; and The Chemistry and Science of Cosmetics, 2nd Ed., Vols 1 and 2 (1962), Van Nostrand Co. Inc., Vols 3 and 4 (1975), Continental Press; both of which are hereby incorporated by reference herein in their entireties.

The cosmetic composition may comprise at least one cosmetically acceptable auxiliary agent. Cosmetically acceptable auxiliary agents include, but are not limited to, carriers, excipients, emulsifiers, surfactants, preservatives, fragrances, perfume oils, thickeners, polymers, gel formers, dyes, absorption pigments, photo protective agents, consistency regulators, antioxidants, antifoams, antistats, resins, solvents, solubility promoters, neutralizing agents, stabilizers, sterilizing agents, propellants, drying agents, opacifiers, cosmetically active ingredients, hair polymers, hair and skin conditioners, graft polymers, water-soluble or dispersible silicone-containing polymers, bleaches, care agents, colorants, tinting agents, tanning agents, humectants, refatting agents, collagen, protein hydrolyzates, lipids, emollients and softeners, tinting agents, tanning agents, bleaches, keratin-hardening substances, antimicrobial active ingredients, photofilter active ingredients, repellant active ingredients, hyperemic substances, keratolytic and keratoplastic substances, antidandruff active ingredients, antiphlogistics, keratinizing substances, active ingredients which act as antioxidants and/or as free-radical scavengers, skin moisturizing or humectants substances, refatting active ingredients, deodorizing active ingredients, sebostatic active ingredients, plant extracts, antierythematous or antiallergic active ingredients, and mixtures thereof. Cosmetic formulations are known in the art. See, for instance, U.S. Patent Application Publication Nos. 2008/0196847 and 2010/0322981.

The combination effect pigment may be added in any tinctorially effective amount to the paint, coating, printing ink, high molecular weight organic material, cosmetic formulation, laser marking, pigment composition, or dry preparation.

The combination effect pigment may be added to such materials as paint, coating, printing ink, high molecular weight organic material, cosmetic formulation, laser marking, pigment composition, or dry preparation at concentrations ranging from 0.0001% wt/wt to 90% wt/wt, 0.001% wt/wt to 80% wt/wt, or 0.01% wt/wt to 50% wt/wt, based on a total weight of the material/formulation.

In some embodiments, a cosmetic formulation may include the combination effect pigment added in an amount from 0.0001% wt/wt to 90% wt/wt based on the total weight of the cosmetic formulation. The cosmetic formulation may further include a cosmetically suitable carrier material ranging from 10% wt/wt to 90% wt/wt. The cosmetically suitable carrier material may be different from water.

In one embodiment, a nail enamel formulation comprises a nail enamel base in an amount ranging from 90.00% wt/wt to 99.00% wt/wt (e.g., 94.00% wt/wt) and a combination effect pigment in an amount ranging from 1.00% wt/wt to 10.00% wt/wt (e.g., 6.00 % wt/wt). In one embodiment, the combination effect pigment is produced according to or similar to any of Examples 1-9 or A1-A9 (e.g., Example A1 or Example A8). For example, an amount of carbon black loading in the combination effect pigment may be adjusted to range from 0.50 wt. % to 2.00 wt. % (e.g., 1.00 wt. %) while maintaining other parameters of the production process. In one embodiment, an amount of carbon black loading in the combination effect pigment ranges from 0.10 wt.% to 1.00 wt. % (e.g., 0.40 wt. %). In one embodiment, the nail enamel formation is prepared by mixing the nail enamel base, and slowly adding the combination effect pigment while avoiding aeration.

In one embodiment, a nail enamel formulation comprises a nail enamel base in an amount ranging from 90.00% wt/wt to 99.00% wt/wt (e.g., 94.00% wt/wt) and a combination effect pigment in an amount ranging from 1.00% wt/wt to 10.00% wt/wt (e.g., 6.00% wt/wt). In one embodiment, the combination effect pigment comprises calcium sodium borosilicate, titanium dioxide, and silica.

In one embodiment, a nail enamel formulation comprises a nail enamel base in an amount ranging from 90.00% wt/wt to 99.00% wt/wt (e.g., 93.06% wt/wt), and an effect pigment comprising calcium sodium borosilicate, titanium dioxide, and silica in an amount ranging from 1.00% wt/wt to 10.00% wt/wt (e.g., 6.00% wt/wt). In one embodiment, the nail enamel formulation further comprises carbon black present in an amount from 0.50% wt/wt to 2% wt/wt (e.g., 0.94% wt/wt). In another embodiment, the nail enamel formulation further comprises iron oxide present in an amount from 0.50% wt/wt to 2% wt/wt (e.g., 0.94% wt/wt).

In one embodiment, a nail enamel formulation comprises a nail enamel base in an amount ranging from 90.00% wt/wt to 99.00% wt/wt (e.g., 94.00% wt/wt), and an effect pigment comprising calcium sodium borosilicate, titanium dioxide, and silica in an amount ranging from 1.00% wt/wt to 10.00% wt/wt (e.g., 5.94% wt/wt). In one embodiment, the nail enamel formulation further comprises carbon black present in an amount from 0.01% wt/wt to 0.10% wt/wt (e.g., 0.06% wt/wt). In another embodiment, the nail enamel formulation further comprises iron oxide present in an amount from 0.01% wt/wt to 0.10% wt/wt (e.g., 0.06% wt/wt).

In one embodiment, an eye shadow formulation comprises a talc as a filler an amount ranging from 50.00% wt/wt to 60.00% wt/wt (e.g., 54.00% wt/wt) and a combination effect pigment in an amount ranging from 35.00% wt/wt to 45.00% wt/wt (e.g., 40.00 % wt/wt). In one embodiment, the combination effect pigment is produced according to or similar to any of Examples 1-9 or A1-A9 (e.g., Example A1 or Example A8). In one embodiment, an amount of carbon black loading in the combination effect pigment ranges from 0.10 wt. % to 1.00 wt. % (e.g., 0.40 wt. %). In one embodiment, an amount of carbon black loading in the combination effect pigment ranges from 0.50 wt.% to 2.00 wt. % (e.g., 1.00 wt. %). In one embodiment, the eye shadow formulation comprises additional constituents, including, but not limited to, hydrogenated polyisobutene as a wet binder (e.g., 2.00% wt/wt), coco-caprylate as a wet binder (e.g., 2.00% wt/wt), polyglyceryl-2 dipolyhydroxystearate as a wet binder (1.00% wt/wt), preservatives (e.g., 1.00% wt/wt, and including one or more of capryl glycol, phenoxyethanol, or hexylene glycol), and combinations thereof.

In one embodiment, an eye shadow formulation comprises a talc as a filler an amount ranging from 50.00% wt/wt to 60.00% wt/wt (e.g., 54.00% wt/wt) and a combination effect pigment in an amount ranging from 35.00% wt/wt to 45.00% wt/wt (e.g., 40.00% wt/wt). In one embodiment, the effect pigment comprises calcium sodium borosilicate, titanium dioxide, and silica. In one embodiment, the eye shadow formulation comprises additional constituents, including, but not limited to, hydrogenated polyisobutene as a wet binder (e.g., 2.00% wt/wt), coco-caprylate as a wet binder (e.g., 2.00% wt/wt), polyglyceryl-2 dipolyhydroxystearate as a wet binder (1.00% wt/wt), preservatives (e.g., 1.00% wt/wt, and including one or more of capryl glycol, phenoxyethanol, or hexylene glycol), and combinations thereof.

In one embodiment, an eye shadow formulation comprises a talc as a filler an amount ranging from 50.00% wt/wt to 60.00% wt/wt (e.g., 54.00% wt/wt) and an effect pigment in an amount ranging from 35.00% wt/wt to 45.00% wt/wt (e.g., 39.60% wt/wt). In one embodiment, the effect pigment comprises calcium sodium borosilicate, titanium dioxide, and silica. In one embodiment, the eye shadow formulation further comprises carbon black in an amount ranging from 0.10% wt/wt to 1.00% wt/wt (e.g., 0.40% wt/wt). In one embodiment, the eye shadow formulation further comprises iron oxide in an amount ranging from 0.10% wt/wt to 1.00% wt/wt (e.g., 0.40% wt/wt). In one embodiment, the eye shadow formulation comprises additional constituents, including, but not limited to, hydrogenated polyisobutene as a wet binder (e.g., 2.00% wt/wt), coco-caprylate as a wet binder (e.g., 2.00% wt/wt), polyglyceryl-2 dipolyhydroxystearate as a wet binder (1.00% wt/wt), preservatives (e.g., 1.00% wt/wt, and including one or more of capryl glycol, phenoxyethanol, or hexylene glycol), and combinations thereof.

### EXAMPLARY EMBODIMENTS

The foregoing embodiments, as well as additional embodiments, are described below.

In one aspect of the present disclosure, a combination effect pigment comprises: a platy substrate; an outer layer disposed above the platy substrate; and carbon black, wherein the carbon black is disposed above the substrate and is entrapped by the outer layer.

In one embodiment, the carbon black is present in a form of particles embedded within the outer layer. In one embodiment, the carbon black is deposited via wet chemical deposition. In one embodiment, the carbon black is deposited via chemical vapor deposition.

In one embodiment, the combination effect pigment further comprises an intermediate layer disposed between the outer layer and the platy substrate. In one embodiment, the carbon black is disposed as a layer between the intermediate layer and the outer layer. In one embodiment, the carbon black is disposed as a layer between the platy substrate and the intermediate layer. In one embodiment, the carbon black is co-deposited with one or more of the intermediate layer or the outer layer.

In one embodiment, the combination effect pigment further comprises a plurality of layers disposed between the platy substrate and the outer layer. In one embodiment, the carbon black is present in a form of particles embedded within one or more of the plurality of layers. In one embodiment, the carbon black is disposed as a layer between any adjacent pair of layers of the combination effect pigment. In one embodiment, at least one layer of the plurality of layers is an optical layer.

In one embodiment, the carbon black is present in the combination effect pigment from 0.01 wt. % to 3.00 wt. % based on a total weight of the uncoated platy substrate. In one embodiment, the carbon black is present in the combination effect pigment from 0.01 wt. % to 0.10 wt. % based on a total weight of the uncoated platy substrate. In one embodiment, the carbon black is present in the combination effect pigment in an amount up to 30.00 wt. % based on a total weight of the uncoated platy substrate. In one embodiment, the carbon black is present in the effect pigment from 10.00 wt. % to 30.00 wt. % based on a total weight of the uncoated platy substrate. In one embodiment, the carbon black is present in a form of a hydrous oxide or hydroxide precipitate.

In one embodiment, the combination effect pigment further comprises a polyvalent cation. In one embodiment, the polyvalent cation is selected from a group consisting of Al, Cr, Ti, Zn, Mg, Zr, Fe, Ce, and Sn. In one embodiment, the polyvalent cation is selected from a group consisting of Al(III), Cr(III), Zn(II), Mg(II), Ti(IV), Zr(IV), Fe(II), Fe(III), Ce(III), and Sn(IV). In one embodiment, the polyvalent cation forms a precipitate layer disposed between the platy substrate and the outer layer. In one embodiment, the polyvalent cation is present in the combination effect pigment from 0.01 wt. % to 1.00 wt. % based on the total weight of the uncoated platy substrate. In one embodiment, a weight ratio of the carbon black to the polyvalent cation ranges from 3:1 to 1:3. In one embodiment, the polyvalent cation has a suitable anionic counterion selected from a group consisting of chloride, nitrate, and sulfate.

In one embodiment, the outer layer is an optical layer. In one embodiment, the optical layer comprises a metal oxide. In one embodiment, the metal oxide is selected from a group consisting of TiO₂, In₂O₃, ZrO₂, Fe₂O₃, Fe₃O₄, Al₂O₃, Cr₂O₃,CeO₂, ZnO, SnO₂, and combinations thereof. In one embodiment, the outer layer comprises SiO₂.

In one embodiment, the combination effect pigment further comprises an intermediate layer disposed between the platy substrate and the outer layer, wherein the outer layer and the intermediate layer are independently selected from an optical layer or a layer comprising SiO₂.

In one embodiment, the carbon black is in direct contact with the platy substrate.

In one embodiment, the platy substrate is selected from a group consisting of iron oxide, synthetic mica, natural mica, basic lead carbonate, flaky barium sulfate, SiO₂, Al₂O₃, TiO₂, glass, ZnO, ZrO₂, SnO₂, BiOCl, chromium oxide, BN, MgO flakes, Si₃N₄, graphite, aluminum, titanium, aluminum alloys, bronzes, iron, and perlite.

In one embodiment, the combination effect pigment further comprises a plurality of layers disposed between the platy substrate and the outer layer, wherein the platy substrate, the carbon black, the plurality of intermediate layers, and the outer layer collectively define a layer structure of the effect pigment selected from a group consisting of: platy substrate/SiO₂/carbon black/SiO₂, platy substrate/carbon black/SiO₂, platy substrate/carbon black/TiO₂, platy substrate/TiO₂/carbon black/TiO₂, platy substrate/carbon black/TiO₂/SiO₂, platy substrate/TiO₂/carbon black/SiO₂, platy substrate/SiO₂/TiO₂/carbon black/Fe₂O₃, platy substrate/TiO₂/carbon black/SiO₂/carbon black/TiO₂, platy substrate/Fe₂O₃/SiO₂/carbon black/ TiO₂/SiO₂, platy substrate/SnO₂/TiO₂/carbon black/TiO₂, platy substrate/TiO₂/SiO₂/carbon black/Fe₂O₃, platy substrate/TiO₂/SiO₂/carbon black/TiO₂, platy substrate/TiO₂/carbon black/SiO₂/Fe₂O₃, platy substrate/TiO₂/carbon black/SiO₂/TiO₂, platy substrate/Fe₂O₃/SiO₂/carbon black/Fe₂O₃, platy substrate/Fe₂O₃/SiO₂/carbon black/TiO₂, platy substrate/Fe₂O₃/carbon black/SiO₂/Fe₂O₃, platy substrate/Fe₂O₃/ carbon black/SiO₂/TiO₂, platy substrate/TiO₂/SiO₂/carbon black/Cr₂O₃, platy substrate/carbon black/Fe₂O₃, platy substrate/TiO₂/SiO₂/TiO₂/carbon black/TiO₂, and platy substrate/TiO₂/SiO₂/carbon black/TiO₂.

In one embodiment, the platy substrate comprises BiOCl. In one embodiment, the carbon black is present as a single layer or is dispersed within one or more adjacent layers of the layer structure.

In another aspect of the present disclosure, a paint, ink-jet, coatings, printing ink, plastic, cosmetic, glaze for ceramics, or glass comprises the combination effect pigment.

In another aspect of the present disclosure, a cosmetic comprises the combination effect pigment. In one embodiment, the cosmetic is in the form of sticks, ointments, creams, emulsions, suspensions, dispersions, powders or solutions. In one embodiment, the cosmetic is a lipstick, mascara preparation, blusher, eye-shadows, foundation, eyeliners, powder or nail varnishes.

In another aspect of the present disclosure, a nail enamel comprises: a nail enamel base; and the combination effect pigment. In one embodiment, the combination effect pigment comprises a layer structure of: platy substrate/TiO₂/SiO₂/TiO₂/carbon black/TiO₂. In one embodiment, the combination effect pigment comprises a layer structure of: platy substrate/TiO₂/SiO₂/carbon black/TiO₂.

In another aspect of the present disclosure, a method of producing a combination effect pigment comprises: depositing carbon black on a platy substrate followed by forming an outer layer over the carbon black; or co-depositing the carbon black and the outer layer on the platy substrate, wherein the outer layer at least partially entraps the carbon black. In one embodiment, the deposition or co-deposition of the carbon black on the platy substrate further comprises depositing or co-depositing a polyvalent cation.

In one embodiment, depositing the carbon black comprises depositing a hydrous oxide or hydroxide precipitate of the carbon black with the polyvalent cation at a given pH. In one embodiment, the method further comprises depositing an intermediate layer prior to depositing the carbon black.

In one embodiment, depositing or co-depositing the carbon black comprises depositing the carbon black via wet chemical deposition or chemical vapor deposition.

In one embodiment, the method further comprises drying the formed combination effect pigment.

The use of the terms "a," "an," "the," and similar referents in the context of describing the materials and methods discussed herein (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the materials and methods and does not pose a limitation on the scope unless otherwise claimed. No language in the specification should be construed as indicating any non-claimed element as essential to the practice of the disclosed materials and methods.

Reference throughout this specification to "one embodiment," "certain embodiments," "one or more embodiments" or "an embodiment" means that a particular feature, structure, material, or characteristic described in connection with the embodiment is included in at least one embodiment of the present disclosure. Thus, the appearances of the phrases such as "in one or more embodiments," "in certain embodiments," "in one embodiment," or "in an embodiment" in various places throughout this specification are not necessarily referring to the same embodiment of the present disclosure. Furthermore, the particular features, structures, materials, or characteristics may be combined in any suitable manner in one or more embodiments.

## Claims

1. A combination effect pigment comprising:
a platy substrate;
an outer layer disposed above the platy substrate, wherein the outer layer is an optical layer; and
carbon black, wherein the carbon black is disposed above the substrate and is entrapped by the outer layer, and wherein the carbon black is present in a form of hydrous oxide or hydroxide precipitate with a polyvalent cation selected from a group consisting of Al, Cr, Ti, Zn, Mg, Zr, Fe, Ce, and Sn.

2. The combination effect pigment of claim 1, wherein the carbon black is present in the combination effect pigment from about 0.01 wt. % to about 3.00 wt. % based on a total weight of the uncoated platy substrate, preferably wherein the carbon black is present in the combination effect pigment from about 0.01 wt. % to about 0.10 wt. % based on a total weight of the uncoated platy substrate.

3. The combination effect pigment of claim 1, wherein the carbon black is present in the combination effect pigment in an amount up to 30.00 wt. % based on a total weight of the uncoated platy substrate.

4. The combination effect pigment of claim 1, wherein a weight ratio of the carbon black to the polyvalent cation ranges from 3:1 to 1:3.

5. The combination effect pigment of claim 1, wherein the optical layer comprises a metal oxide, selected from a group consisting of TiO₂, In₂O₃, ZrO₂, Fe₂O₃, Fe₃O₄, Al₂O₃, Cr₂O₃, CeO₂, ZnO, SnO₂, and combinations thereof.

6. The combination effect pigment of claim 1, wherein the outer layer is a SiO₂ layer.

7. The combination effect pigment of claim 1, wherein the carbon black is in direct contact with the platy substrate.

8. The combination effect pigment of claim 1, wherein the platy substrate is selected from a group consisting of iron oxide, synthetic mica, natural mica, basic lead carbonate, flaky barium sulfate, SiO₂, Al₂O₃, TiO₂, glass, ZnO, ZrO₂, SnO₂, BiOCl, chromium oxide, BN, MgO flakes, Si₃N₄, graphite, aluminum, titanium, aluminum alloys, bronzes, iron, and perlite.

9. The combination effect pigment of claim 1, further comprising:
a plurality of layers disposed between the platy substrate and the outer layer, wherein the platy substrate, the carbon black, the plurality of intermediate layers, and the outer layer collectively define a layer structure of the effect pigment selected from a group consisting of:
platy substrate/SiO₂/carbon black/SiO₂,
platy substrate/carbon black/SiO₂,
platy substrate/carbon black/TiO₂,
platy substrate/TiO₂/carbon black/TiO₂,
platy substrate/carbon black/TiO₂/SiO₂,
platy substrate/TiO₂/carbon black/SiO₂,
platy substrate/SiO₂/TiO₂/carbon black/Fe₂O₃,
platy substrate/TiO₂/carbon black/SiO₂/carbon black/TiO₂,
platy substrate/Fe₂O₃/SiO₂/carbon black/ TiO₂/SiO₂,
platy substrate/SnO₂/TiO₂/carbon black/TiO₂,
platy substrate/TiO₂/SiO₂/carbon black/Fe₂O₃,
platy substrate/TiO₂/SiO₂/carbon black/TiO₂,
platy substrate/TiO₂/carbon black/SiO₂/Fe₂O₃,
platy substrate/TiO₂/ carbon black/SiO₂/TiO₂,
platy substrate/Fe₂O₃/SiO₂/carbon black/Fe₂O₃,
platy substrate/Fe₂O₃/SiO₂/carbon black/TiO₂,
platy substrate/Fe₂O₃/carbon black/SiO₂/Fe₂O₃,
platy substrate/Fe₂O₃/ carbon black/SiO₂/TiO₂,
platy substrate/TiO₂/SiO₂/carbon black/Cr₂O₃,
platy substrate/carbon black/Fe₂O₃,
platy substrate/TiO₂/SiO₂/TiO₂/carbon black/TiO₂, and
platy substrate/TiO₂/SiO₂/carbon black/TiO₂.

10. A paint, ink-jet, coatings, printing ink, plastic, cosmetic, glaze for ceramics, or glass comprising the combination effect pigment of claim 1.

11. A cosmetic comprising the combination effect pigment of claim 1.

12. A nail enamel comprising:
a nail enamel base; and
the combination effect pigment of claim 1.

13. The nail enamel of claim 12, wherein the combination effect pigment comprises a layer structure of: platy substrate/TiO₂/SiO₂/TiO₂/carbon black/TiO₂.

14. The nail enamel of claim 12, wherein the combination effect pigment comprises a layer structure of: platy substrate/TiO₂/SiO₂/carbon black/TiO₂.

## Patentansprüche

1. Kombinationseffektpigment, umfassend:
ein plättchenförmiges Substrat;
eine über dem plättchenförmigen Substrat angeordnete äußere Schicht, wobei es sich bei der äußeren Schicht um eine optische Schicht handelt; und
Ruß, wobei der Ruß über dem Substrat angeordnet ist und durch die äußere Schicht eingeschlossen ist und wobei der Ruß in Form eines Oxidhydrat-oder Hydroxid-Präzipitats mit einem mehrwertigen Kation aus der Gruppe bestehend aus Al, Cr, Ti, Zn, Mg, Zr, Fe, Ce und Sn vorliegt.

2. Kombinationseffektpigment nach Anspruch 1, wobei der Ruß in dem Kombinationseffektpigment in einer Menge von etwa 0,01 Gew.-% bis etwa 3,00 Gew.-%, bezogen auf das Gesamtgewicht des unbeschichteten plättchenförmigen Substrats, vorliegt, vorzugsweise wobei der Ruß in dem Kombinationseffektpigment in einer Menge von etwa 0,01 Gew.-% bis etwa 0,10 Gew.-%, bezogen auf das Gesamtgewicht des unbeschichteten plättchenförmigen Substrats, vorliegt.

3. Kombinationseffektpigment nach Anspruch 1, wobei der Ruß in dem Kombinationseffektpigment in einer Menge von bis zu 30,00 Gew.-%, bezogen auf das Gesamtgewicht des unbeschichteten plättchenförmigen Substrats, vorliegt.

4. Kombinationseffektpigment nach Anspruch 1, wobei das Gewichtsverhältnis von Ruß zu mehrwertigem Kation im Bereich von 3:1 bis 1:3 liegt.

5. Kombinationseffektpigment nach Anspruch 1, wobei die optische Schicht ein Metalloxid aus der Gruppe bestehend aus TiO₂, In₂O₃, ZrO₂, Fe₂O₃, Fe₃O₄, Al₂O₃, Cr₂O₃, CeO₂, ZnO, SnO₂ und Kombinationen davon umfasst.

6. Kombinationseffektpigment nach Anspruch 1, wobei es sich bei der äußeren Schicht um eine SiO₂-Schicht handelt.

7. Kombinationseffektpigment nach Anspruch 1, wobei der Ruß in direktem Kontakt mit dem plättchenförmigen Substrat steht.

8. Kombinationseffektpigment nach Anspruch 1, wobei das plättchenförmige Substrat aus der Gruppe bestehend aus Eisenoxid, synthetischem Glimmer, natürlichem Glimmer, basischem Bleicarbonat, plättchenförmigem Bariumsulfat, SiO₂, Al₂O₃, TiO₂, Glas, ZnO, ZrO₂, SnO₂, BiOCl, Chromoxid, BN, MgO-Plättchen, Si₃N₄, Graphit, Aluminium, Titan, Aluminiumlegierungen, Bronzen, Eisen und Perlit ausgewählt ist.

9. Kombinationseffektpigment nach Anspruch 1, ferner umfassend:
mehrere Schichten, die zwischen dem plättchenförmigen Substrat und der äußeren Schicht angeordnet sind, wobei das plättchenförmige Substrat, der Ruß, die mehreren Zwischenschichten und die äußere Schicht zusammen eine Schichtstruktur des Effektpigments aus der Gruppe bestehend aus
plättchenförmigem Substrat/SiO₂/Ruß/SiO₂,
plättchenförmigem Substrat/Ruß/SiO₂,
plättchenförmigem Substrat/Ruß/TiO₂,
plättchenförmigem Substrat/TiO₂/Ruß/TiO₂,
plättchenförmigem Substrat/Ruß/TiO₂/SiO₂,
plättchenförmigem Substrat/TiO₂/Ruß/SiO₂,
plättchenförmigem Substrat/SiO₂/TiO₂/Ruß/Fe₂O₃,
plättchenförmigem Substrat/TiO₂/Ruß/SiO₂/Ruß/TiO₂,
plättchenförmigem Substrat/Fe₂O₃/SiO₂/Ruß/TiO₂/SiO₂,
plättchenförmigem Substrat/SnO₂/TiO₂/Ruß/TiO₂,
plättchenförmigem Substrat/TiO₂/SiO₂/Ruß/Fe₂O₃,
plättchenförmigem Substrat/TiO₂/SiO₂/Ruß/TiO₂,
plättchenförmigem Substrat/TiO₂/Ruß/SiO₂/Fe₂O₃,
plättchenförmigem Substrat/TiO₂/Ruß/SiO₂/TiO₂,
plättchenförmigem Substrat/Fe₂O₃/SiO₂/Ruß/Fe₂O₃,
plättchenförmigem Substrat/Fe₂O₃/SiO₂/Ruß/TiO₂,
plättchenförmigem Substrat/Fe₂O₃/Ruß/SiO₂/Fe₂O₃,
plättchenförmigem Substrat/Fe₂O₃/Ruß/SiO₂/TiO₂,
plättchenförmigem Substrat/TiO₂/SiO₂/Ruß/Cr₂O₃,
plättchenförmigem Substrat/Ruß/Fe₂O₃,
plättchenförmigem Substrat/TiO₂/SiO₂/TiO₂/Ruß/TiO₂ und
plättchenförmigem Substrat/TiO₂/SiO₂/Ruß/TiO₂ definieren.

10. Anstrichmittel, Tintenstrahl, Beschichtungen, Druckfarbe, Kosmetikum, Glasur für Keramik oder Glas, umfassend das Kombinationseffektpigment nach Anspruch 1.

11. Kosmetikum, umfassend das Kombinationseffektpigment nach Anspruch 1.

12. Nagellack, umfassend:
eine Nagellackgrundlage und
das Kombinationseffektpigment nach Anspruch 1.

13. Nagellack nach Anspruch 12, wobei das Kombinationseffektpigment die Schichtstruktur plättchenförmiges Substrat/TiO₂/SiO₂/TiO₂/Ruß/TiO₂ aufweist.

14. Nagellack nach Anspruch 12, wobei das Kombinationseffektpigment die Schichtstruktur plättchenförmiges Substrat/TiO₂/SiO₂/Ruß/TiO₂ aufweist.

## Revendications

1. Pigment à effet combinatoire, comprenant :
un substrat lamellaire ;
une couche externe disposée au-dessus du substrat lamellaire, où la couche externe est une couche optique ; et
du noir de carbone, où le noir de carbone est disposé au-dessus du substrat et est piégé par la couche externe, et où le noir de carbone est présent sous la forme d'un précipité d'oxyde ou d'hydroxyde hydraté avec un cation polyvalent choisi dans le groupe constitué par Al, Cr, Ti, Zn, Mg, Zr, Fe, Ce, et Sn.

2. Pigment à effet combinatoire selon la revendication 1, dans lequel le noir de carbone est présent dans le pigment à effet combinatoire selon d'environ 0,01% en poids à environ 3,00% en poids sur la base d'un poids total du substrat lamellaire non revêtu, préférablement où le noir de carbone est présent dans le pigment à effet combinatoire selon d'environ 0,01% en poids à environ 0,10% en poids sur la base d'un poids total du substrat lamellaire non revêtu.

3. Pigment à effet combinatoire selon la revendication 1, dans lequel le noir de carbone est présent dans le pigment à effet combinatoire selon une quantité allant jusqu'à 30,00% en poids sur la base d'un poids total du substrat lamellaire non revêtu.

4. Pigment à effet combinatoire selon la revendication 1, dans lequel un rapport pondéral du noir de carbone au cation polyvalent se trouve dans la plage allant de 3:1 à 1:3.

5. Pigment à effet combinatoire selon la revendication 1, dans lequel la couche optique comprend un oxyde métallique choisi dans le groupe constitué par TiO₂, In₂O₃, ZrO₂, Fe₂O₃, Fe₃O₄, Al₂O₃, Cr₂O₃, CeO₂, ZnO, SnO₂, et des combinaisons de ceux-ci.

6. Pigment à effet combinatoire selon la revendication 1, dans lequel la couche externe est une couche de SiO₂.

7. Pigment à effet combinatoire selon la revendication 1, dans lequel le noir de carbone se trouve en contact direct avec le substrat lamellaire.

8. Pigment à effet combinatoire selon la revendication 1, dans lequel le substrat lamellaire est choisi dans le groupe constitué par l'oxyde de fer, le mica synthétique, le mica naturel, le carbonate de plomb basique, le sulfate de baryum lamellaire, SiO₂, Al₂O₃. TiO₂, le verre, ZnO, ZrO₂, SnO₂, BiOCl, l'oxyde de chrome, BN, des paillettes de MgO, Si₃N₄, le graphite, l'aluminium, le titane, les alliages d'aluminium, les bronzes, le fer et la perlite.

9. Pigment à effet combinatoire selon la revendication 1, comprenant en outre :
une pluralité de couches disposées entre le substrat lamellaire et la couche externe, où le substrat lamellaire, le noir de carbone, la pluralité de couches intermédiaires, et la couche externe, définissent collectivement une structure en couches du pigment à effet choisie dans le groupe constitué par :
substrat lamellaire/SiO₂/noir de carbone/SiO₂;
substrat lamellaire/noir de carbone/SiO₂ ;
substrat lamellaire/noir de carbone/TiO₂ ;
substrat lamellaire/TiO₂/noir de carbone/TiO₂ ;
substrat lamellaire/noir de carbone/TiO₂/SiO₂ ;
substrat lamellaire/TiO₂/noir de carbone/SiO₂ ;
substrat lamellaire/SiO₂/TiO₂/noir de carbone/Fe₂O₃ ;
substrat lamellaire/TiO₂/noir de carbone/SiO₂/noir de carbone/TiO₂ ;
substrat lamellaire/Fe₂O₃/SiO₂/noir de carbone/TiO₂/SiO₂ ;
substrat lamellaire/SnO₂/TiO₂/noir de carbone/TiO₂ ;
substrat lamellaire/TiO₂/SiO₂/noir de carbone/Fe₂O₃ ;
substrat lamellaire/TiO₂/SiO₂/noir de carbone/TiO₂ ;
substrat lamellaire/TiO₂/noir de carbone/SiO₂/Fe₂O₃ ;
substrat lamellaire/TiO₂/noir de carbone/SiO₂/TiO₂ ;
substrat lamellaire/Fe₂O₃/SiO₂/noir de carbone/Fe₂O₃ ;
substrat lamellaire/Fe₂O₃/SiO₂/noir de carbone/TiO₂ ;
substrat lamellaire/Fe₂O₃/noir de carbone/SiO₂/Fe₂O₃ ;
substrat lamellaire/Fe₂O₃/noir de carbone/SiO₂/TiO₂ ;
substrat lamellaire/TiO₂/SiO₂/noir de carbone/Cr₂O₃ ;
substrat lamellaire/noir de carbone/Fe₂O₃ ;
substrat lamellaire/TiO₂/SiO₂/TiO₂/noir de carbone/TiO₂ ; et
substrat lamellaire/TiO₂/SiO₂/noir de carbone/TiO₂.

10. Peinture, jet d'encre, revêtements, encre d'impression, matière plastique, produit cosmétique, glaçure pour céramiques, ou verre, comprenant le pigment à effet combinatoire selon la revendication 1.

11. Produit cosmétique, comprenant le pigment à effet combinatoire selon la revendication 1.

12. Vernis à ongles, comprenant
une base de vernis à ongles ; et
le pigment à effet combinatoire selon la revendication 1.

13. Vernis à ongles selon la revendication 12, dans lequel le pigment à effet combinatoire comprend une structure en couches de type : substrat lamellaire/TiO₂/SiO₂/TiO₂/noir de carbone/TiO₂.

14. Vernis à ongles selon la revendication 12, dans lequel le pigment à effet combinatoire comprend une structure en couches de type : substrat lamellaire/TiO₂/SiO₂/noir de carbone/TiO₂.
